Europäisches Patentamt

European Patent Office

Office européen des brevets

(19)

(11) **EP 1 223 848 B1**

(12) **EUROPÄISCHE PATENTSCHRIFT**

(45) Veröffentlichungstag und Bekanntmachung des Hinweises auf die Patenterteilung:
**13.09.2006 Patentblatt 2006/37**

(21) Anmeldenummer: **01965240.3**

(22) Anmeldetag: **31.08.2001**

(51) Int Cl.:
*A61B 3/10* (2006.01)     *A61B 3/12* (2006.01)

(86) Internationale Anmeldenummer:
**PCT/EP2001/010070**

(87) Internationale Veröffentlichungsnummer:
**WO 2002/017775 (07.03.2002 Gazette 2002/10)**

(54) **SYSTEM ZUR BERÜHRUNGSLOSEN VERMESSUNG DER OPTISCHEN ABBILDUNGSQUALITÄT EINES AUGES**

SYSTEM FOR MEASURING THE OPTICAL IMAGE QUALITY OF AN EYE IN A CONTACTLESS MANNER

SYSTEME DE MESURE SANS CONTACT DE LA QUALITE DE REPRODUCTION OPTIQUE D'UN OEIL

(84) Benannte Vertragsstaaten:
**AT BE CH CY DE DK ES FI FR GB GR IE IT LI LU MC NL PT SE TR**

(30) Priorität: **31.08.2000 DE 10042751**

(43) Veröffentlichungstag der Anmeldung:
**24.07.2002 Patentblatt 2002/30**

(73) Patentinhaber: **CARL ZEISS JENA GmbH 07745 JENA (DE)**

(72) Erfinder:
• HELLMUTH, Thomas
  73430 Aalen (DE)
• TAN, Lieng-Chuong
  73430 Aalen (DE)

(56) Entgegenhaltungen:
WO-A-00/28884        WO-A-00/43730
WO-A-95/33970        WO-A-96/35100
US-A- 5 329 321      US-A- 5 975 699

**Beschreibung**

Die Erfindung betrifft ein System zur berührungslosen Vermessung der optischen Abbildungsqualität eines Auges mit einem Interferometer über welches wenigstens ein Lichtimpuls mit einer kurzen Kohärenzlänge von einer Lichtquelle in das Auge eingekoppelt wird.

Nach Operationen im Bereich der Augenlinse und der Hornhaut (Cornea), wie beispielsweise Grauer-Star-Operationen oder refraktiven Eingriffen an der Cornea, können am Sehsystem des Auges optische Abweichungen, sogenannte Aberrationen, auftreten, die mit Hilfe sphärischer oder astigmatischer Linsen bzw. Zylinderlinsen nicht korrigierbar sind. Ein weiteres Problem dieser Aberrationen liegt darin, daß sie sich mit steigendem Pupillendurchmesser drastisch erhöhen und so insbesondere die Nachtsichtfähigkeit ernsthaft einschränken.

Daher ist es wichtig, diese Aberrationen meßtechnisch zu erfassen, um entsprechende Korrekturmaßnahmen, möglichst schon während der Operation, einleiten zu können. Im Falle einer Grauen-Star-Operation, bei welcher die ursprüngliche Linse entfernt und durch eine künstliche Linse ersetzt wird, ist es außerdem sehr wichtig die Länge des Auges zu messen, um eine angemessene künstliche Ersatzlinse auszuwählen, welche der Augenlänge angepaßt ist und so ein gutes Sehen ermöglicht.

Da diese Vermessungen der Abbildungsqualität des Auges einen Einfluß sowohl auf die Linse im Zusammenspiel mit der Augenlänge als auch auf die Aberrationen haben, und schon während und/oder kurz nach der Operation erfolgen sollen, sind im Lichte der Qualitätssicherung dieser Verfahrensweisen nur kontaktlose bzw. berührungslose Verfahren anwendbar, um Gewebereizungen und Infektionen zu vermeiden. Die bisher übliche Messung der Augenlänge mit Ultraschall scheidet also aus, da diese das Aufsetzen eines Ultraschallkopfes auf die Cornea erforderlich macht.

Der Artikel "Optical Coherence Tomography" von A. Fercher, im "Journal of Biomedical Optics, Vol. 1, No. 2, April 1996, pp. 157-173" beschreibt ein Verfahren zur Analyse der Augenlänge im kontaktlosen Modus.

Über ein zweiarmiges Interferometer, welches beispielsweise ein Michelson-Interferometer sein kann, wird Licht als kontinuierliches Licht oder wenigstens in Form von kurzen Lichtimpulsen bzw. Wellenzügen in das zu vermessende Auge eingekoppelt. Durch eine vorgegebene Armlängendifferenz des Interferometers, welche in etwa der zu erwartenden Länge des menschlichen Auges, welche üblicherweise zwischen 24 mm und 28 mm angesiedelt ist, entspricht, kann erreicht werden, daß es im Bereich einer Sensorik zu einer Interferenz einer von der Cornea stammenden Lichtreflexion und einer von der Netzhaut (Retina) stammenden Lichtreflexion kommt. Ein Reflektor im Bereich des Interferometers kann dabei über eine Meßeinrichtung, einen sogenannten Scanner, so weit meßbar verfahren werden, bis es zu dem gewünschten Interferenzmuster zwischen dem retinalen und cornealen Reflex kommt. Aus dem dafür erforderlichen meßbaren Verfahrweg ergibt sich zusammen mit der bekannten Ausgangsstellung und der bekannten vorgehaltenen Armlängendifferenz des Interferometers eine Größe, welche exakte Rückschlüsse auf die Länge des vermessenen Auges, also den Abstand zwischen Oberfläche der Cornea und Oberfläche der Retina, ermöglicht.

Bei diesem Ansatz kann allerdings in nachteiliger Weise nur die reine Länge des Auges gemessen werden.

In der US 5,975,699 ist ein Verfahren und eine Vorrichtung beschrieben, welches gleichzeitig die Länge des Auges und den Brechungsfehler mißt.

Diese Vorrichtung, welche in der Lage ist, die Länge des Auges und den Brechungsfehler gleichzeitig zu messen, koppelt über ein Michelson-Interferometer mit einer vorgegebenen Längendifferenz zwischen den beiden sich kreuzenden optischen Armen und über einen weiteren Strahlteiler Licht in das Innere des Auges ein. Die cornealen und retinalen Reflexe gelangen dann über diesen Strahlteiler teilweise ungenutzt in das Interferometer, der andere für die Messung genutzte Teil gelangt über eine optische Abbildungseinrichtung zu einem Gitter, an welchem es zu einer spektralen Zerlegung des Lichts kommt. Danach kommt es zu einer spektrometrischen Auswertung dieses Lichts hinsichtlich des Spektrums, zur Ermittlung des refraktiven Fehlers bzw. des Brechungsfehlers des Auges, und zur Auswertung der durch die Interferenzen, aufgrund der unter-schiedlichen Weglängen des Interferometers erzeugten Intensitätsschwankungen des cornealen und des retinalen Reflexes, um so die Länge des Auges zu ermitteln.

Den beiden oben genannten Verfahren ist es gemeinsam, daß sie nicht in der Lage sind, höhere Abweichungen des Sehsystems meßtechnisch zu erfassen. Zusätzlich wird bei beiden Verfahren ein externes Interferometer mit einer Armlängendifferenz angewandt, die der optischen Länge des zu untersuchenden Auges entspricht. Ein prinzipgemäßes Merkmal des Interferometers besteht darin, daß fünfzig Prozent des Lichtes am Strahlteiler verlorengehen und durch die somit erfolgenden Abschwächung der Intensität des eingeleiteten Lichts und der Reflexionen die gute Erkennbarkeit der Signale verringert wird. Wird ein Michelson-Interferometer eingesetzt, so ist dies außerdem umständlich in der Ausrichtung und Handhabung.

In dem Artikel "Messplatz zur Bestimmung der monochromatischen Aberration des menschlichen Auges" von P. Mierdel, H.E. Krinke, W. Wiegand, M. Kaemmerer, T. Seiler, in "Der Ophthalmologe", Vol. 94, Juni 1997, pp. 441-445 ist ein Verfahren zur Analyse der optischen Abweichungen eines Auges beschrieben.

Dabei wird ein regelmäßiges Muster von Lichtpunkten auf dem Augenfundus in dem Bereich der Retina des zu untersuchenden Auges erzeugt, indem über eine Lochmaske erzeugte Lichtstrahlen durch eine Sammellinse in einem kleinen Abstand vor der Retina fokusiert werden. Dadurch kommt es dann bei einem idealen Auge zu einem gleichmäßigen Muster von Lichtpunkten auf der Retina. Bei einem Auge mit entsprechenden Aberrationen im Bereich der Linse oder der Cornea kommt es dagegen zu einem verzerrten Muster von Lichtpunkten auf der Retina. Über entsprechende optische Abbildungseinrichtungen wird dann ein Zwischenbild des retinalen Lichtpunktmusters erzeugt und über ein Kameraobjektiv auf einem lichtempfindlichen CCD-Sensor abgebildet. Die Abbildung des Lichtpunktmusters wird verzerrt, wenn das Sehsystem optische Aberrationen aufweist. Diese Aberrationen können numerisch analysiert werden..Das Ergebnis wird als eine Liste von Gewichtsfaktoren von Zernika-Polynomen ausgegeben, womit sich ein "Wellenfrontaberrationsgebirge" modellieren läßt.

Ein weiteres Verfahren wird in dem Artikel "Objective measurement of wave aberrations of the human eye with the use of a Hartmann-Shack wave front sensor" von J. Liang, B. Grimm, S. Goelz, J.F. Bille, im "J. Opt. Soc. Am. A", Vol. 11, no. 7, July, 1994 beschrieben.

Mittels einer Reflexion von eingeleitetem Licht wird eine sekundäre Lichtquelle im Bereich der Retina auf dem Augenhintergrund des zu untersuchenden Auges erzeugt. Das Lichtstrahlenbündel der retinalen Reflexion wird dann mit einer Linsenanordnung, einem sogenannten Linsenarray, auf ein CCD-Ziel konzentriert. Das Lichtstrahlenbündel, das aus den Pupillen austritt, besteht bei einem emmetropischen, also einem idealen bzw. gesunden Auge ohne Aberrationen, aus parallelen Strahlen. Somit stellen bei einem emmetropischen Auge die durch das Linsenarray gebündelten Strahlen ein regelmäßiges gitterähnliches Lichtpunktmuster dar. Bei Aberrationen des Sehsystems weisen einzelne Strahlen aus dem Strahlenbündel, aufgrund der Abweichung der Wellenfront des Lichtes, das aus den Augenpupillen austritt, Abweichungen von ihrer idealen Richtung bzw. Parallelität auf. Bei einem Auge mit Aberrationen weicht das Lichtpunktmuster also von dem regelmäßigen Muster des emmetropen Auges ab. Diese Abweichungen können dann numerisch analysiert werden, um Gewichtsfaktoren von Zernike-Polynomen zu erhalten.

Die beiden zuletzt genannten Verfahren machen es möglich, Aberrationen des Sehsystems zu messen. Sie sind in nachteiliger Weise jedoch darauf beschränkt, daß mit ihnen lediglich diese Aberrationen und nicht die Länge des menschlichen Auges gemessen werden kann.

Aus WO 96/35100 ist eine Vorrichtung bekannt, welche einem rotierenden Glasblock, mit welchem die optische Weglänge des Referenzarms periodisch verändert wird, aufweist Bei der Rotation des Glasbloks ändert sich dabei sowohl die geometrische als auch die optische Weglänge. Eine Messung der Augenlänge ist mit dieser Lösung nicht möglich, da dazu der Glasblock entsprechend groß zu dimensionieren wäre und damit nicht mehr schnell genug rotieren könnte um die Störeinflüsse der Sakkadenbewegungen des Auges zu eliminieren.

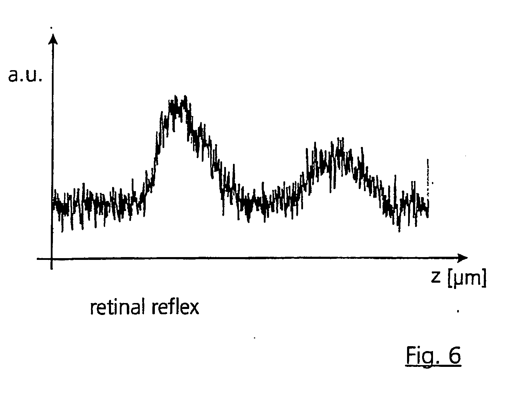

Die US 5,329,321 offenbart eine Vorrichtung, welche die zur Messung der Augenlänge notwendige Änderung der optischen Weglänge entweder durch eine spiralförmige Spiegelfläche (Fig. 6) oder ein spiralförmiges Keilrad (Fig. 13) realisiert. Auch diese optischen Elemente sind damit so groß zu dimensionieren, dass eine schnelle Rotation nicht möglich ist und somit Messfehler durch Augenbewegungen nicht verhindert werden können.

Es ist daher die Aufgabe der Erfindung, ein System zur berührungslosen Vermessung der optischen Abbildungsqualität eines Auges vor, während und nach Operationen im Bereich der Linse und/oder der Hornhaut zu schaffen, welches möglichst viele Informationen liefert, um, über die Operation oder nachträgliche Korrekturmaßnahmen, ein möglichst ideales Sehen bei wenigstens annähernd allen auftretenden Lichtverhältnissen zu ermöglichen.

Erfindungsgemäß wird diese Aufgabe dadurch gelöst, daß die Abbildungsqualität eines Auges mit wenigstens einem Lichtimpuls mit einer kurzen Kohärenzlänge vermessen wird, welcher über ein Interferometer in das Auge eingekoppelt wird. Die optische Weglänge wenigstens eines Arms des Interferometers wird dabei zur Messung der Länge des Auges variiert, bis in einem Detektor ein typisches Interferenzmuster zwischen einer Reflexion von der Hornhaut und einer Reflexion von der Netzhaut des Auges auftritt. Zusammen mit einem bekannten Wegstück der Variation der optischen Weglänge, erlaubt dies Rückschlüsse auf die Länge des Auges. Die Variation der optischen Weglänge erfolgt über das Einbringen von wenigstens teilweise transparenten Elementen und durch wenigstens ein definiert bewegbares Element des Interferometers in wenigstens einem Lichtweg des Interferometers.

Bei dem erfindungsgemäßen System kann die Messung der Länge des Auges direkt in dem Interferometer ablaufen. Dabei wird erfindungsgemäß die optische Weglänge wenigstens eines Arms des Interferometers über wenigstens teilweise optisch transparente Elemente und durch wenigstens ein definiert bewegbares Element, je nach Ausgestaltung der Erfindung kann es sich dabei entweder um einen Reflektor oder um einen Sensor handeln, was für die Funktionsweise des interferometerischen Messens gleichbedeutend ist, variiert. Durch das Einbringen der optisch wenigstens teilweise transparenten Elemente, welche in einer besonders günstigen Ausführungsform der Erfindung beispielsweise als Zylinder aus Polymethylmethacrylat (PMMA) ausgebildet sein können, wird die optische Länge entsprechend der Lauflänge des Lichts und des Brechungsindex in dem wenigstens teilweise transparenten Materials der Elemente verändert. Um eine Interferenz festzustellen, muß daher der Reflektor je nach eingebrachtem optischen Element nur um ein sehr kleines Wegstück bewegt werden, bis das Interferenzmuster auftritt.

So kann in besonders vorteilhafter Weise eine sehr schnelle Messung durchgeführt werden, da die gesamte zu vermessende Länge des zu vermessenden Auges, welche im allgemeinen zwischen 24 mm und 28 mm variiert, in mehrere, beispielsweise vier, Gruppen eingeteilt werden kann. Durch das wenigstens teilweise transparente Element, welches für jede der Gruppen in einer anderen Länge ausgebildet ist, kann erreicht werden, daß für jede der zu vermessenden Längengruppe der Reflektor jeweils nur um 1 mm bewegt werden muß. Findet der Sensor bei dieser Bewegung ein passendes Interferenzmusters, so wird in der nächstliegenden Gruppe mit dem nächstkürzeren bzw. -längeren wenigstens teilweise transparenten Element die gleiche Messung nochmals durchgeführt. Dadurch ergibt sich der Vorteil, daß die Messung selbst, welche prinzipiell nur während des Verstellen des Reflektors stattfindet, in ihrem zeitlichen Ablauf verkürzt werden kann, wodurch Meßfehler, welche beispielsweise durch eine relative Bewegung zwischen dem Auge als zu messendes Objekt und dem Interferometer auftreten könnten, durch die Verkürzung der Zeit entscheidend minimiert werden.

In einer besonders vorteilheften Ausgestaltung und Weiterbildung der Erfindung werden die Reflexionen von der Retina über eine optische Abbildungseinrichtung zu einer Einrichtung zur Erfassung der Aberrationen der Wellenfronten in Mydriasis, also in den nicht parachsialen bzw. achsfernen Bereichen des Auges, weitergeleitet.

In einer besonders günstigen Weiterbildung dieser Ausgestaltung der Erfindung kann diese Einrichtung als Hartmann-Shack-Sensor ausgebildet sein.

Diese Kombination aus interferometrischer Messung der Länge des Auges und Analyse der Aberrationen, welche durch die Cornea und gegebenenfalls auch teilweise durch die Linse, überwiegend in den bezüglich der optischen Achse des Auges parachsialen Bereichen auftreten können, hat entscheidende Vorteile. Ein erster gravierender Vorteil entsteht durch den Aufbau selbst, welcher in der Lage ist, über einen einzigen von einer Lichtquelle erzeugten Bezugspunkt, welcher durch eine entsprechende Reflexion des in das Auge eingekoppelten Lichts erzeugt wird, beide Messungen durchzuführen. Die Vergleichbarkeit der Meßwerte und die Möglichkeit einer Korrelation zwischen den Meßwerten bezüglich der Aberrationen und bezüglich der Länge des Auges, welche beide auf dem gleichen Bezugspunkt basieren, wird geschaffen und die Qualität und damit der praktische Nutzen der Meßergebnisse für den Operateur und den Patienten kann so verbessert werden.

Außerdem entsteht durch die Kombination ein Instrument zur Vermessung aller entscheidenden Größen bezüglich der Abbildungsqualität des Auges, welches aufgrund seiner kontaktlosen Ausführung auch während einer Operation, z.B. beim Einbringen eines Intraokularlinsen-Implantats, eine entsprechende Überwachung, Qualitätssicherung und gegebenenfalls Korrektur der vorgenommenen Veränderungen durch den Operateur erlaubt.

Das erfindungsgemäße System ist in vorteilhafter Weise sehr schnell und einfach anzuwenden und liefert sowohl Werte bezüglich der Augenlänge, welche bei der Auswahl der Linse für das Implantat hilfreich sind, und kann gleichzeitig Aufschluß über eventuelle entstehende Aberrationen im Bereich der Cornea geben, so daß damit durch die Auswahl einer entsprechenden Linse bei der Operation gegebenenfalls erzeugte Spannungen in der Cornea, welche diese Aberrationen aufgrund von Verformungen oder dergleichen hervorrufen können, umgehend entgegengewirkt werden kann, indem eine entsprechende Anpassung des Implantats oder eine andere geeignete Korrekturmaßnahme durch den Operateur sofort ergriffen wird.

In diesem speziellen Anwendungsfall, der Messung der Abbildungsqualität des Auges während der Operation, entsteht der entscheidende Vorteil, daß beim Erkennen eines Fehlers sofort reagiert werden kann und keine weitere Operation erforderlich ist, wie dies beispielsweise bei einer Vermessung, welche erst nach dem Ausheilen der durch die Operation entstehenden Wunden erfolgt, gegebenenfalls zur Korrektur erforderlich wäre.

Das Risiko einer weiteren erforderlichen Operation wird gesenkt, was insbesondere für ältere Menschen, welche als die hauptsächliche Zielgruppe für Grauer-Star-Operation anzusehen ist, eine entscheidende Erleichterung bedeutet, da diese bei jeder Form von Operationen sicherlich als Patientengruppe mit erhöhtem Risiko anzusehen sind.

Weitere vorteilhafte Ausgestaltungen der Erfindung ergeben sich aus den restlichen Unteransprüchen und dem nachfolgend anhand der Zeichnung dargestellten Ausführungsbeispiel.

Es zeigt:

Fig. 1    eine Ausführungsform eines Systems zur Vermessung der optischen Abbildungsqualität eines Auges;

Fig. 2    eine detaillierte Darstellung eines Rades aus Fig. 1;

Fig. 3    eine prinzipmäßige Darstellung eines Wellenfrontfehlers bei einem Auge;

Fig. 4    azimuthale und radiale Differentiale von an einer Pupille austretenden Strahlen;

Fig. 5    eine Hüllkurve eines Interferenzsignals von der Hornhaut; und

Fig. 6    eine Hüllkurve eines Interferenzsignals von der Netzhaut.

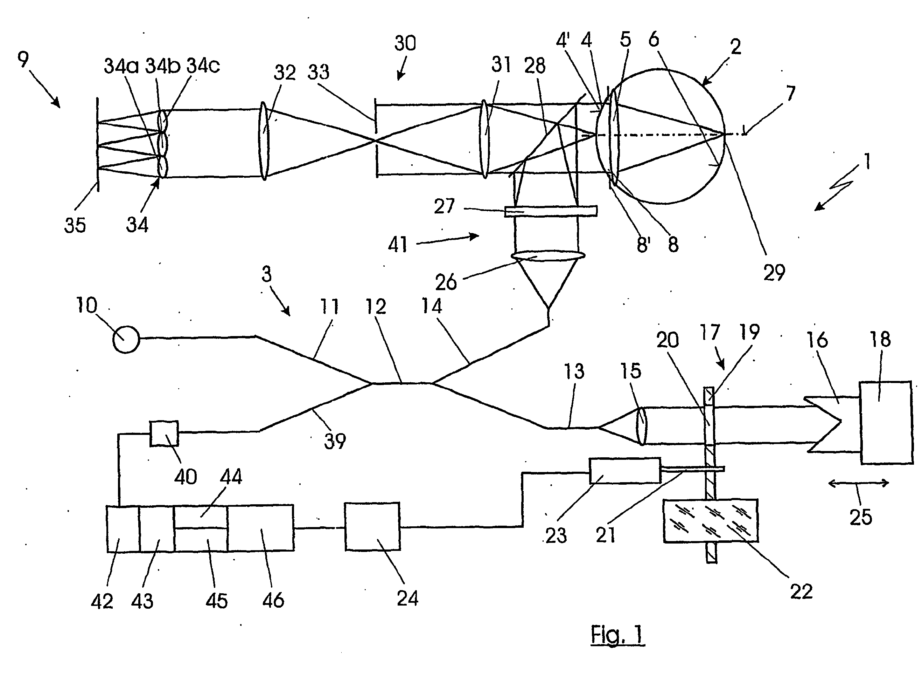

In Fig. 1 ist eine mögliche Ausführungsform des Systems 1 zur berührungslosen Vermessung der optischen Abbildungsqualität eines Auges 2 mit einem Interferometer 3 dargestellt. Das System 1 soll vorwiegend zur Vermessung des menschlichen Auges 2 vor, während und nach operativen Eingriffen im Bereich der Hornhaut (Cornea) 4 und der Linse 5 eingesetzt werden. Ein Einsatz des Systems 1 zur Vermessung ähnlich aufgebauter Augen 2 anderer Säugetiere ist jedoch prinzipiell auch denkbar, da das System 1 ohnehin selbsttätig arbeitet und keine Rückmeldung des mit dem Auge 2 Sehenden erforderlich macht. Dies prädestiniert das System 1 auch für den Einsatz während Operationen, bei denen der mit dem Auge 2 sehende Patient im allgemeinen zumindest teilweise unter Betäubung steht, so daß eine Rückmeldung hier gegebenenfalls schwierig wäre.

Das System 1 ist zur gleichzeitigen Messung von Aberrationen und von einer Länge L des Auges 2 auf kontaktlose Art, also ohne daß eine Sonde oder dergleichen das Auge 2 berührt, vorgesehen.

Unter der Länge L des Auges 2 versteht man dabei den Abstand zwischen der Oberfläche 4' der Cornea 4, dem sogenannten Epithelium anterius, und dem Augenhintergrund (Fundus) mit der Netzhaut (Retina) 6.

Die Aberrationen sind Abweichungen der Lichtstrahlen in dem Auge 2 von den idealen Wegen dieser Lichtstrahlen. Diese Aberrationen treten überwiegend beim Lichtdurchtritt durch die Cornea 4 auf und werden durch Verformungen in der Cornea 4 verursacht. Besonders kritisch sind hierbei die nicht parachsialen Bereiche der Cornea, also die von der optischen Achse 7 (axis opticus) des Auges 2 entfernt liegenden Bereiche der Cornea 4, welche nur bei weit geöffneter Regenbogenhaut (Iris) 8', also bei einer großen Pupille 8 in Mydriasis, z.B. bei schlechten Lichtverhältnissen, zum Tragen kommen.

Wie in Fig. 1 erkennbar, besteht das System 1 aus dem Interferometer 3, welches als faseroptisches Interferometer 3 ausgebildet ist, einer Einrichtung 9 zur Erfassung der Aberrationen in den Wellenfronten, welche als Hartmann-Shack-Sensor 9 ausgebildet ist, und einer Lichtquelle 10, welche Licht mit einer kurzen Kohärenzlänge emittiert.

In dem bevorzugten Ausführungsbeispiel gemäß Fig. 1 ist die Lichtquelle 10 als Superluminiszenzdiode (SLD) ausgebildet, welche ihre Strahlung direkt in eine optische Faser 11 einkoppelt, welche als sogenannte Anschlußfaser bezeichnet wird. Die Faser 11 wird an einem ihrer Enden mit dem Faserende eines 3dB-Kopplers 12 verbunden, der die Strahlung in zwei Fasern 13 und 14 aufteilt. Die Strahlung vom Ende der Faser 13 wird in einer Linse 15 gesammelt und trifft auf einen Reflektor 16. Das Licht wird von dem Reflektor 16 in das Ende der Faser 13 und damit auch in den 3dB-Koppler 12 zurückreflektiert.

In der nachfolgenden Beschreibung werden der Reflektor 16 und die als Sammellinse ausgebildete Linse 15 als Referenzarm 17 des faseroptischen Interferometers 3 bezeichnet. Der Reflektor 16 ist auf einem angetriebenen Halteelement 18 angebracht, das vorzugsweise linear vorwärts und rückwärts bewegbar ist. Außerdem weist der Referenzarm 17 des faseroptischen Interferometers 3 ein sich drehendes Rad 19 zwischen dem Reflektor 16 und der Linse 15 auf, welches in Fig. 1 in einem Querschnitt prinzipmäßig angedeutet ist.

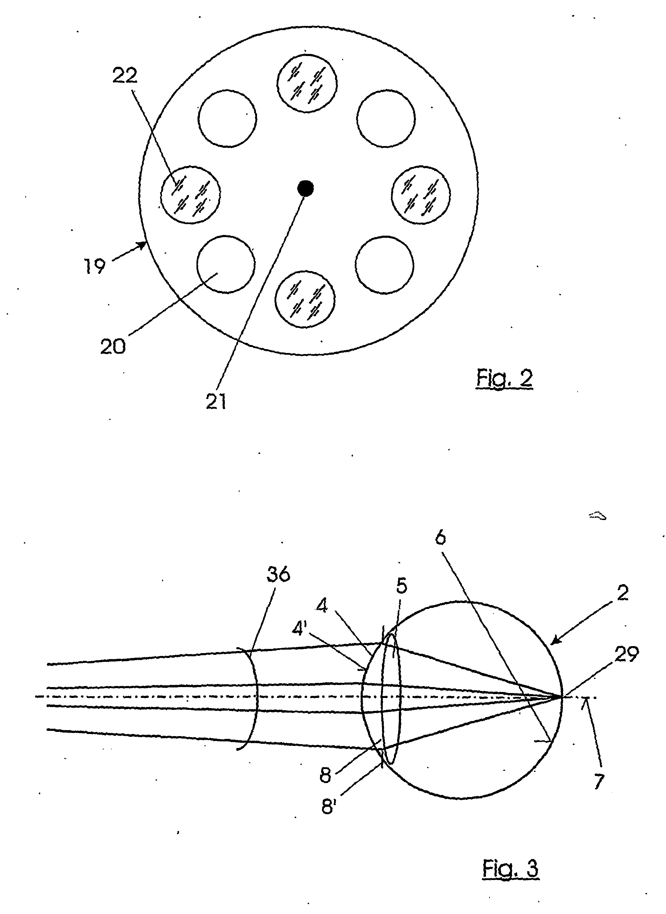

Wie in Fig. 2 dargestellt, sind in das Rad 19 mehrere Blendenöffnungen 20 eingebracht, deren Mittelpunkte auf einem Kreis liegen, in dessen Mittelpunkt eine zentrale Achse 21 des Rads 19 angeordnet ist. Jede zweite der Blendenöffnungen 20 enthält ein zumindest teilweise optisch transparentes Element 22, welches hier jeweils als ein durchsichtiger Zylinder 22, der vorzugsweise aus PMMA (Polymethylacrylat) besteht, ausgebildet ist. Die durchsichtigen Zylinder 22 haben dabei unterschiedliche Längen $l$, wobei zumindest einer der Zylinder 22 mit der Länge $l = L/(n-1)$ ausgebildet ist, wobei n den Brechungsindex des Zylinders 22 aus PMMA (z.B. n=1, 336) darstellt. $L = n \cdot \Lambda$ ist dabei die optische Länge $L$ eines normalen Auges 2 der Länge $\Lambda$.

Wird dieser durchsichtige Zylinder 22 der Länge $l$ nun in den optischen Weg bzw. den Lichtstrahl des Referenzarms 17 eingebracht , so verändert sich dessen optische Armlänge. Idealerweise werden dementsprechend die Längen $l$ der einzelnen Zylinder 22 so gewählt, daß über alle vorhandenen Zylinder 22 die Armlänge in einzelnen Stufen so verkürzt werden kann, daß mit den Verkürzungen alle üblicherweise auftretenden Längen $L$ eines Auges 2 abgedeckt werden können. Der Referenzarm 17 des Interferometers 3 kann also gegenüber den anderen Armen des Interferometers 3 um die zu erwartende Länge $L$ des Auges 2 verkürzt werden.

Das Rad 19 wird durch einen Schrittmotor 23 angetrieben. Die Frequenz der Antriebsschaltung einer Steuerung/Regelung 24 des Schrittmotors 23 wird so gewählt, daß die Drehung mit einer periodischen linearen Bewegung 25 des Reflektors 16 synchronisiert wird. Daher ändert sich die optische Armlänge des Referenzarms 17 des faseroptischen Interferometers 3 periodisch zwischen $L_0$ und $L_0 + L$, mit der Referenzarmlänge $L_0$ ohne eingebrachten Zylinder 22.

Das Licht von dem Ende der Faser 14 wird von einer weiteren Linse 26 gesammelt und über ein diffraktives optisches Element (DOE) 27 und einen Strahlteiler 28 zu dem Auge 2 des Patienten geleitet. Das diffraktive optische Element 27 ist so ausgelegt, daß die erste Beugungsordnung auf die Oberfläche 4' der Cornea 4 fokusiert wird. Um sicherzustellen, daß zumindest während des Zeitpunkts der eigentlichen Messung keine Verfälschungen der Messung auftreten, muß gewährleistet werden, daß die Oberfläche 4' der Cornea 4 des Auges 2, welches vermessen wird, in einem definierten gleichbleibenden Abstand zu dem System 1 oder zumindest zu dem diffraktiven optischen Element 27 verbleibt. Dies kann beispielsweise über eine entsprechende Stütze für Kinn und Stirn des Patienten erfolgen, welche direkt mit dem System 1 gekoppelt ist, wie dies von Vorrichtungen zur Vermessung der Sehschärfe und dergleichen an sich bekannt ist.

Die Beugungswirksamkeit des diffraktiven optischen Elements 27 wird so gewählt, daß nur etwa fünf Prozent des auftreffenden Lichtes in die erste Beugungsordnung gebeugt wird. Das Sehsystem des Auges 2, bestehend aus der Cornea 4 und der Linse 5, konzentriert die nullte Beugungsordnung des diffraktiven optischen Elements 27 im Hintergrund des Auges 2 auf die Retina 6 und erzeugt durch Reflexion eine sekundäre Lichtquelle 29 auf der Retina 6.

Das von dieser sekundären Lichtquelle 29 auf der Retina 6 ausgehende Licht, welches nur die in etwa $10^{-4}$-fache Intensität des eingestrahlten Lichts hat, wird bei einem emmetropischen Auge 2 durch das Sehsystem des Auges 2 in im wesentlichen parallelen Strahlen gesammelt, welche zu dem Strahlteiler 28 gelangen. Ein Teil der gesammelten Strahlung wird von dem Strahlteiler 28 abgelenkt und zu dem diffraktiven optischen Element 27 geführt. Aufgrund der geringen Beugungswirksamkeit des diffraktiven optischen Elements 27 durchdringt das Licht von der sekundären Lichtquelle 29 auf der Retina 6 das diffraktive optische Element 27 nahezu unbeeinflußt und wird durch die Linse 26 in dem Ende der Faser 14 gesammelt.

Der auf die Oberfläche 4' der Cornea 4 fokusierte Strahl wird von dieser teilweise reflektiert. Der Reflexionsgrad der Cornea 4 beträgt dabei etwa vier Prozent. Weitere fünf Prozent des reflektierten Lichts von der Cornea 4 werden durch das diffraktive optische Element 27 in einem Fokus gesammelt. Sie gehen somit für das System 1 gewollt verloren, da nur der parallel an der Linse 26 ankommende Teil des Lichts in das Ende der Faser 14 konzentriert wird. Die Intensität dieses durch die Linse 26 in das Ende der Faser 14 gesammelten Lichtflusses des reflektierten Lichtes von der Cornea 4 beträgt somit ebenfalls nur das $10^{-4}$-fache der Intensität des Eingangslichtes. Daher handelt es sich bei dem von der Cornea 4 reflektierten Licht um ein Licht derselben Intensität, wie bei dem Licht von der sekundären Lichtquelle 29 auf der Retina 6. Die beiden Lichtstrahlen vergleichbarer Intensität werden dann mittels der Sammellinse 26 in das Ende der Faser 14 fokusiert.

Der andere Teil des Lichts, der auf den Strahlteiler 28 auftrifft, durchdringt diesen und gelangt über eine optische Abbildungseinrichtung 30 zu der Einrichtung 9 zur Erfassung der Aberrationen in den Wellenfronten, welche hier als Hartmann-Shack-Sensor 9 ausgebildet ist. Durch den Strahlteiler 28 wird somit erreicht, daß die sekundäre Lichtquelle 29 auf der Retina 6 gleichzeitig für die Messung der Länge L des Auges 2 und die Messung der Aberrationen verwendet werden kann.

Der Hartmann-Shack-Sensor 9 weist in der bevorzugten, in Fig. 1 dargestellten, Ausführungsform die Abbildungseinrichtung 30 mit zwei Sammellinsen 31 und 32, einer Blende 33, einem Linsenarray 34 sowie einem Detektorfeld 35, vorzugsweise aus CCD-Sensoren, auf.

Die Sammellinse 31 fokusiert die von der sekundären Lichtquelle 29 stammenden Lichtstrahlen im Bereich der Blende 33. Das von der Oberfläche 4' der Cornea 4 reflektierte Licht ist divergierend und wird von der Sammellinse 31 parallelisiert. Diese parallelen Strahlen des von der Cornea 4 reflektierten Lichts werden dann durch die Blende 33 abgeschattet. Die Blende 33 fungiert somit als Strahlensperre, die fast das gesamte von der Cornea 4 reflektierte Licht eliminiert.

Das Licht der sekundären Lichtquelle 29 auf der Retina 6 ist im Bereich der Blende 33 fokusiert und kann die Blende 33 so ungehindert durchdringen. Es wird dann von der Sammellinse 32 zu einem parallelen Strahl gesammelt, welcher auf das Linsenarray 34 auftrifft. Die optische Abbildungseinrichtung 30 mit den zwei Sammellinsen 31 und 32 sowie der Blende 33 bildet also die Pupille 8 des Auges 2 auf der Ebene des Linsenarrays 34 ab. Jede einzelne der Linsen 34a, 34, 34c, von denen hier drei Stück exemplarisch angedeutet sind, des Linsenarrays 34 fokusiert die Lichtstrahlenbündel, die auf jeder der Linsen 34a, 34, 34c auftreffen, auf das Detektorfeld 35 mit dem wenigstens einen CCD-Sensor. Dabei liegt eine sicherlich sinnvolle Größe des Linsenarrays 34 bei ca. 5x5 bis 20x20 einzelnen Linsen 34a, 34b, 34c, ... .

Bei einem emmetropischen Auge 2 ohne Aberrationen sind die von den einzelnen Linsen 34a, 34b, 34c, erzeugten Brennpunkte abstandsgleich und bilden ein regelmäßiges Lichtpunktemuster. Bei einem Auge 2 mit Aberrationen ist das Lichtpunktemuster entsprechend verzerrt.

Nachfolgend ist nun ausführlich beschrieben, wie mit dem in Fig. 1 und Fig. 2 aufgezeigten System 1 die entsprechenden Vermessungen am Auge 2 erfolgen. Zuerst soll die Vermessung der Aberrationen des Auges 2 beschrieben werden:

In Fig. 3 ist zu erkennen, daß die Strahlen des Strahlenbündels, das aus der Pupille 8 des Auges 2 austritt, als die Normalen einer Fläche 36 betrachtet werden können. Die Fläche 36 ist als gesamtes gesehen eine Kugel, wenn die Strahlen einen gemeinsamen Kreuzungspunkt aufweisen. Bei Aberrationen handelt es sich dagegen bei den als gesamtes gesehenen Flächen 36 um einen asphärischen Körper. Dabei ist es prinzipiell so gedacht, daß der über das Interferometer 3 in das Auge 2 eingekoppelte Lichtstrahl einen so geringen Durchmesser hat, daß dieser durch die Mitte der Pupille 8 in das Auge 2 eindringt.. Dadurch kann sichergestellt werden, daß der Strahl beim Eindringen in das Auge 2 keine Verzerrungen oder Veränderungen erfährt, da im allgemeinen in den achsnahen bzw. parachsialen Bereichen der Cornea 4 und der Linse 5 keine Aberrationen auftreten. Dies ist auch besonders günstig, da so eventuell auftretende Aberrationen keinerlei Einfluß auf die Messung der Länge $L$ des Auges 2 über das Interferometer 3 haben, welche nachfolgend noch näher beschrieben wird.

Die über das Sehsystem wieder aus dem Auge 2 austretenden Lichtstrahlen gelangen unter anderem auch durch den nicht achsnahen Bereich der Pupille 8 in Mydriasis; so daß hier eventuell auftretende Aberrationen, welche insbesondere die Nachtsichtfähigkeit des Patienten nachteilig beeinflussen könnten, über den Hartmann-Shack-Sensor 9 erfaßt werden können.

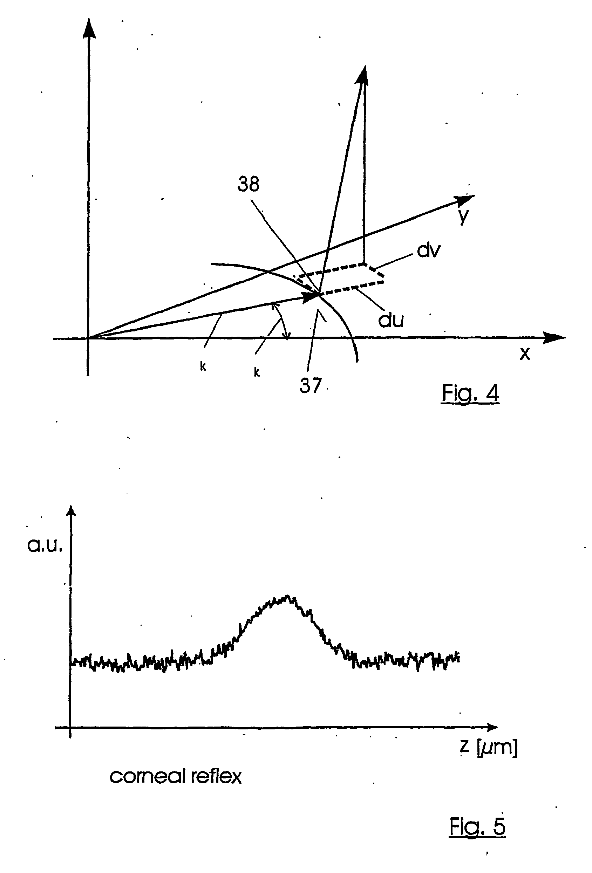

In Fig. 3 ist dies prinzipmäßig dadurch angedeutet, daß an der Fläche 36 im Randbereich eine stärkere Abweichung von der idealen Fläche in Form eines Kugelabschnitts angedeutet ist, als dies in den parachsialen bzw. achsnahen Bereichen der Fall ist. Die Funktion, die die Fläche 36 beschreibt, kann durch Zernike-Polynome beschrieben werden, wobei es sich um Funktionen in den Polarkoordinaten $\rho$ und $\varphi$ in der Pupillenebene 37 gemäß Fig. 4 handelt. Die Koordinate p ist normiert und wird am Mittelpunkt der Pupille 8 als 0, und am Rand der Pupille 8 als 1 angenommen. Betrachtet man zur Vereinfachung nur die Aberrationen der niedrigsten Ordnung, die sogenannten Seidel-Aberrationen, benötigt man acht Zernike-Polynome, um die asphärische Fläche zu beschreiben:

$$f(\rho, \varphi) = \sum_{i=1}^{8} a_i Z_i(\rho, \varphi)$$

mit

$$Z_1 = \rho \cos \varphi$$

$$Z_2 = \rho \sin \varphi$$

$$Z_3 = 2\rho^2 - 1$$

$$Z_4 = \rho^2 \cos 2\varphi$$

$$Z_5 = \rho^2 \sin 2\varphi$$

$$Z_6 = (3\rho^2 - 2) \rho \cos \varphi$$

$$Z_7 = (3\rho^2 - 2)\rho \sin \varphi$$

$$Z_8 = 6\rho^4 - 6\rho^2 + 1$$

wobei $Z_1$ und $Z_2$ die Neigung, $Z_3$ die Defokussierung, $Z_4$ und $Z_5$ den Astigmatismus, $Z_6$ und $Z_7$ den Asymmetriefehler, und $Z_8$ die sphärische Aberration darstellen.

Die unbekannten Koeffizienten $a_i$ können bestimmt werden, wenn die vollständigen Differentiale der Radialverlagerung du und der Azimuthalverlagerung dv gemäß Fig. 4 an mindestens vier Punkten 38, von denen einer exemplarisch dargestellt ist, mit den Koordinaten $(\rho_k, \varphi_k)$, $k = 1, 2, 3, 4$ bekannt sind. Die Differentiale du, dv stehen in direktem Zusammenhang mit der Neigung der Fläche $f(\rho, \varphi)$:

$$grad_\rho\, f(\rho, \varphi) = \frac{\partial\, f(\rho, \varphi)}{\partial \rho} = du(\rho, \varphi) \qquad\qquad (2)$$

$$grad_\varphi \; f(\rho,\varphi) \;=\; \frac{1}{\rho} \; \frac{\partial \; f(\rho_k,\varphi_k)}{\partial\rho} = dv(\rho,\varphi) \qquad (3)$$

Diese zwei Gleichungen (2), (3) gelten für die vier Strahlen, welche die Pupille 8 an den Koordinaten $(\rho_k,\varphi_k)$, $k$ = 1, 2, 3, 4 schneiden. Somit existieren insgesamt acht Gleichungen, mit denen sich die acht unbekannten Koeffizienten $a_i$ bestimmen lassen.

Wenn Aberrationen höherer Ordnungen bestimmt werden sollen, so müssen weitere Zernike-Polynome höherer Ordnung miteinbezogen werden. Dann sind natürlich mehr als vier Strahlen mit ihren Differentialen notwendig, um alle Koeffizienten $a_i$ bestimmen zu können.

Grundlegend läßt sich für das System 1 auch jedes andere Wellenfrontanalyseprinzip verwenden, wie beispielsweise eine Interferometer-Testvorrichtung. Dabei müßte man allerdings Nachteile in Kauf nehmen, da es Interferometer-Testvorrichtungen an dynamischer Bandbreite und Robustheit mangelt, wie allgemein bekannt ist.

Die nachfolgend aufgeführten Ausführungen widmen sich der Beschreibung, wie die Länge $L$ des Auges 2 mittels des in Fig. 1 und Fig. 2 beschriebenen System 1 vermessen werden kann:

In dem System 1 wird die Strahlung von der sekundären Lichtquelle 29 auf der Retina 6 des Auges 2 und die vom Reflektor 16 reflektierte Strahlung durch den 3dB-Koppler 12 überlagert. Die Strahlung gelangt durch eine Faser 39 zu einem Detektor 40, welcher in besonders günstiger Weise ebenfalls als CCD-Sensor ausgebildet ist, und wird von diesem registriert. Gleichzeitig führt der sich bewegende Reflektor 16 zu einer zeitweiligen Veränderung der Länge des Referenzarms 17.

Bei der Lichtquelle 10 handelt es sich, wie bereits erwähnt, um eine Superluminiszenzdiode (SLD) mit einer Kohärenzlänge in der Ordnung von 20 $\mu$m. An dem Detektor 40 wird ein moduliertes Signal erkannt, wenn die Länge des Referenzarms 17 innerhalb der Kohärenzlänge der Lichtquelle 10 gleich der Länge eines nachfolgend als Prüfarm 41 bezeichneten Bereichs des Systems 1 ist. Der Prüfarm 41 umfaßt dabei die Faser 14, die Linse 26, das diffraktive optische Element 27 sowie den Strahlteiler 28 und das in seiner Länge $L$ zu vermessende Auge 2.

Wenn die Länge des Referenzarms 17 gleich der Länge des Prüfarms 41 mit der sekundären Lichtquelle 29 auf der Retina 6 als Endpunkt ist, wird am Detektor 40 ein Signal (retinal reflex) erkannt, welches prinzipiell wie in Fig. 6 dargestellt aussieht. Wenn dagegen die Länge des Referenzarms 17 gleich der Länge des Prüfarms 41 mit der Reflexion von der Oberfläche 4' der Cornea 4 als Endpunkt ist, wird ein Signal (corneal reflex) erkannt, welches prinzipiell dem entspricht, das in Fig. 5 dargestellt ist.

Die Länge des Referenzarms 17 verändert sich außerdem durch das sich drehende Rad 19 mit den PMMA-Zylindern 22 und den Blendenöffnungen 20. Dabei wird die Länge $l$ der PMMA-Zylinder 22 mit $l = L/(n-l)$ ausgewählt, wobei $L$ mit $L = n \cdot l$ die optische Länge des normalen Auges 2 ist. Es gilt:

$l$     Länge des PMMA-Zylinders 22

$L$     Länge des Auges 2

$s$     optische Weglänge mit PMMA-Zylinder 22

$s_0$     optische Weglänge ohne PMMA-Zylinder 22

$$\Delta s = s - s_0 = n \cdot l - l = l \cdot (n-1)$$

Wird nun die optische Weglängendifferenz $\Delta s$ so gewählt, daß sie in derselben Größenordnung $(\Delta s = L)$ wie die Länge $L$ des Auges 2 liegt, so gilt:

$$L = l \cdot (n-1) \quad bzw. \quad l = L/(n-1)$$

Bei den üblicherweise auftretenden Längen des normalen Auges 2 eines Patienten von $L$ = 24 mm bis $L$ = 28 mm, werden bei einem Brechungsindex des Materials PMMA von $n$ = 1,336 im dargestellten Ausführungsbeispiel Zylinder 22 in der Größenordnung von 70 mm bis 85 mm Verwendung finden.

Die retinalen und cornealen Signale an dem Detektor 40 werden mit einem Analog-Digital (AD)-Wandler 42 aufgezeichnet. Diese Datenerfassung mit Detektor 40 und AD-wandler, 42 wird mit einem von der Steuerung/Regelung 24 des Schrittmotors 23 gesendeten Startsignal synchronisiert, wenn der Lichtstrahl in dem Referenzarm 17 einen der PMMA-Zylinder 22 auf dem sich drehenden Rad 19 durchdringt. Der Lichtstrahl in dem Referenzarm 17 durchdringt die PMMA-Zylinder 22 und die Blendenöffnungen 22 in einem Zeitintervall T mit derselben Dauer, da die Durchmesser der PMMA-Zylinder 22 und der Blendenöffnungen 20 gleich sind.

Der Reflektor 16 des Referenzarms 17 führt während dieses Zeitintervalls T, in dem der Lichtstrahl des Referenzarms 17 den PMMA-Zylinder 22 durchdringt, mehrmals die periodische Vor- und Rückwärtsbewegung 25 aus. Das dabei vom Detektor 40 kommende Signal wird über den AD-Wandler 42 und einen Duplexer 43 auf ein RAM-Register 44 geschaltet, wenn sich der Reflektor 16 in Vorwärtsbewegung befindet, während ein Adreßzeiger des RAM-Registers 44 aufwärts gezählt wird. Bewegt sich der Reflektor 16 dagegen rückwärts, so wird der Adreßzeiger des RAM-Registers 44 abwärts gezählt.

Alle Werte, die erfaßt wurden, während der Lichtstrahl einen der PMMA-Zylinder 22 durchdrungen hatte, werden an dem jeweiligen Adreßplatz des RAM-Registers 44 zusammengefaßt und gemittelt. Die Beeinflussung der Messung durch Störsignale und Rauschen kann damit minimiert werden.

Sobald der Lichtstrahl des Referenzarms 17 eine der Blendenöffnungen 20 des sich drehenden Rades 19 durchdringt, wird dasselbe Erfassungs- und Mittelwertbildungsverfahren wiederholt. Die Daten werden jedoch in einem weiteren RAM-Register 45 zusammengefaßt.

Die einhüllende Funktion der Interferogramme, wie in Fig. 5 und 6 dargestellt, kann dann mit der Hilbert-Transformation berechnet werden. Wenn $f(t)$ die Signalfunktion ist, liefert die Hilbert-Transformation $H$ den imaginären Teil des analytischen Signals $\tilde{f}$ mit dem echten Teil $f(t)$:

$$\tilde{f}(t) = f(t) + j \cdot H \cdot \{f(t)\}$$

Die einhüllende Funktion $u(t)$ des Signals lautet:

$$u(t) = \left| f(t) \right| = \sqrt{f^2(t) + (H\{f(t)\})^2}$$

Eine mögliche Bandbreite d der Bewegung 25 des Reflektors 16 wird dabei so gewählt, daß $L \pm \Delta/2$ die erwartete Augenlängenverteilung der Patientenpopulation, welche im allgemeinen zwischen 24mm und 28mm liegt, abdeckt. Der Lageort des Mittelpunktes des cornealen Reflexes und des retinalen Reflexes wird aus Datenvektoren, welche während des Zeitintervalls $T$ aufgezeichnet wurden, in einer zentralen alles berechnenden und steuernden Datenverarbeitungseinheit 46 errechnet. Das mit dem Reflex von der Retina 6 korrespondierende Signal gemäß Fig. 6 besteht aus zwei Reflexen. Der erste, größere dieser Reflexe stammt vom epithelialen Pigment, dem sogenannten pars pigmentosa, das sich unmittelbar hinter der Empfängerschicht der Retina 6 befindet. Der zweite, kleinere der Reflexe stammt dagegen von der pars nervosa, einer Nervenschicht, die unmittelbar vor der Empfängerschicht der Retina 6 angeordnet ist. Für die Messung der Augenlänge $L$ kann der Mittelwert der Positionen der zwei Spitzen für die Position der Retina 6 verwendet werden, da die beiden Schichten pars pigmentosa und pars nervosa sehr dicht beieinander angeordnet sind und ihre mittlere Lage praktisch der Lage der Retina 6 entspricht. Das mit dem Reflex von der Cornea 4 korrespondierende Signal besteht aus nur einem Reflex von der Oberfläche 4' der Cornea 4, dem sogenannten epithelium anterius, so daß die Position der Cornea 4 direkt verwendet werden kann.

Die Augenlänge $L$ errechnet sich dann aus der Differenz zwischen den zwei Positionen:

**Patentansprüche**

1. System zur berührungslosen Vermessung der optischen Abbildungsqualität eines Auges (2) mit einem Interferometer (3), wobei das System so gestaltet ist, dass es folgendes Messverfahren realisiert: Wenigstens ein Lichtimpuls wird mit einer kurzen Kohärenzlänge von einer Lichtquelle (10) in das Auge (2) eingekoppelt, wobei die optische Weglänge wenigstens eines Arms des Interferometers (3) zur Messung der Länge (L) des Auges (2) variiert wird, bis in einem Detektor (40) ein typisches Interferenzmuster zwischen einer Reflexion von der Hornhaut (4) und einer Reflexion von der Netzhaut (6) des Auges (2) auftritt, welches zusammen mit einem bekannten Wegstück (As) der Variation der optischen Weglänge Rückschlüsse auf die Länge (L) des Auges (2) erlaubt, wobei die Variation der optischen Weglänge (As) über das Einbringen von ersten optisch wenigstens teilweise transparenten Elementen (22) und durch wenigstens ein zweites definiert bewegbares Element des Interferometers (16) in dem jeweils wenigstens einen Arm (17) des Interferometers (3) erfolgt und wobei die optische Länge des ersten wenigstens teilweise transparenten Elementes (22) an die optische Länge des Auges (2) angepasst ist.

2.  System nach Anspruch 1,
    **dadurch gekennzeichnet, daß**
    das definiert bewegbare Element des Interferometers (3) als Reflektor (16) ausgebildet ist.

3.  System nach Anspruch 2,
    **dadurch gekennzeichnet, daß**
    das Bewegen (25) des Reflektors (16) und das Einbringen der optisch zumindest teilweise transparenten Elemente (22) in den Lichtweg des selben Arms (17) des Interferometers (3) erfolgt.

4.  System nach Anspruch 1, 2 oder 3,
    **dadurch gekennzeichnet, daß**
    das Interferometer (3) als faseroptisches Interferometer ausgebildet ist.

5.  System nach einem der Ansprüche 1 bis 4,
    **dadurch gekennzeichnet, daß**
    die Reflexion der Netzhaut (6) über eine optische Abbildungseinrichtung (30) zu einer Einrichtung (9) zur Erfassung der Aberrationen der Wellenfronten in Mydriasis des Auges (2) gelangt.

6.  System nach Anspruch 5,
    **dadurch gekennzeichnet, daß**
    die Einrichtung zur Erfassung der Aberrationen der Wellenfronten in Mydriasis des Auges (2) als Hartmann-Shack-Sensor (9) ausgebildet ist.

7.  System nach einem der Ansprüche 1 bis 6,
    **dadurch gekennzeichnet, daß**
    der Reflektor (16) mittels einer Antriebseinrichtung (18) in seiner linearen Position meßbar bewegt wird.

8.  System nach einem der Ansprüche 1 bis 7,
    **dadurch gekennzeichnet, daß**
    die optisch wenigstens teilweise transparenten Elemente als Zylinder (22) aus Polymethylmethacrylat, d.h. PMMA, ausgebildet sind.

9.  System nach Anspruch 8,
    **dadurch gekennzeichnet, daß**
    die Zylinder (22) auf einem wenigstens annähernd senkrecht zur optischen Achse des Reflektors angeordneten Rad (19) angebracht sind.

10. System nach Anspruch 9,
    **dadurch gekennzeichnet, daß**
    jeweils einer der Zylinder (22) auf dem Rad (19) in der Art einer Revolvertrommel wahlweise in den Lichtweg einschwenkbar ist.

11. System nach Anspruch 9 oder 10,
    **dadurch gekennzeichnet, daß**
    das Rad (19) von einem Schrittmotor (23) angetrieben wird.

12. System nach Anspruch 9, 10 oder 11,
    **dadurch gekennzeichnet, daß**

auf dem Rad (19) abwechselnd Blendenöffnungen (20) und Zylinder (22) angeordnet sind.

13. System nach einem der Ansprüche 1 bis 12,
**dadurch gekennzeichnet, daß**
eine Süperluminiszenzdiode, d.h. SLD, als Lichtquelle (10) dient.

14. System nach einem der Ansprüche 4 bis 13,
**dadurch gekennzeichnet, daß**
das Licht aus einem Ende einer Faser (14) des Interferometers (3) über eine optische Abbildungseinrichtung in das Auge (2) gelangt.

15. System nach Anspruch 14,
**dadurch gekennzeichnet, daß**
die Abbildungseinrichtung wenigstens eine Linse (26) aufweist.

16. System nach Anspruch 14 oder 15,
**dadurch gekennzeichnet, daß**
die Abbildungseinrichtung wenigstens ein diffraktives optisches Element (27) aufweist.

17. System nach Anspruch 14, 15 oder 16,
**dadurch gekennzeichnet, daß**
die Abbildungseinrichtung wenigstens einen Strahlteiler (28) zum Einkoppeln der Einrichtung (9) zur Erfassung der Aberrationen der Wellenfronten aufweist.

18. System nach Anspruch 16,
**dadurch gekennzeichnet, daß**
das diffraktive optische Element (27) in der Art ausgebildet ist, daß die erste Beugungsordnung auf die Oberfläche (4') der Hornhaut (4) fokusiert wird.

19. System nach einem der Ansprüche 6 bis 18,
**dadurch gekennzeichnet, daß**
der Hartmann-Shack-Sensor (9) ein Linsenarray (34) und ein Detektorfeld (35) aufweist.

20. System nach Anspruch 19,
**dadurch gekennzeichnet, daß**
das Detektorfeld (35) mit CCD-Sensoren ausgebildet ist.

21. System nach einem der Ansprüche 1 bis 20,
**gekennzeichnet durch**
wenigstens eine elektronische Datenverarbeitungseinrichtung (46/24) zur Erfassung und Auswertung der Meßdaten sowie zur Steuerung/Regelung des Verfahrweges des Reflektors (16).

**Claims**

1. System for contact-free measurement of the optical imaging quality of an eye (2) by an interferometer (3), wherein the system is so designed that it realizes the following measuring method: at least one light pulse is launched with a short coherent length by a light source (10) into the eye (2), wherein the optical path length of at least one arm of the interferometer (3) for measuring the length (L) of the eye (2) is varied until in a detector (40) a typical interference pattern between a reflection from the cornea (4) and a reflection from the retina (6) of the eye (2) arises, which together with a known path piece (As) of the variation of the optical path length allows conclusions to be drawn about the length (L) of the eye (2), wherein the variation of the optical path length (As) is effected by the introduction of first, optically at least partially transparent elements (22) and by means of at least one second element of the interferometer (3) that is movable in a defined manner in the, in each case, at least one arm (17) of the interferometer (3) and wherein the optical length of the first, at least partially transparent element (22) is adapted to the optical length of the eye (2).

2. System according to claim 1,

EP 1 223 848 B1

**characterized in that**
the element of the interferometer (3) that is movable in a defined manner takes the form of a reflector (16).

3.  System according to claim 2,
    **characterized in that**
    the movement (25) of the reflector (16) and the introduction of the optically at least partially transparent elements (22) are effected into the light path of the same arm (17) of the interferometer (3).

4.  System according to claim 1, 2 or 3,
    **characterized in that**
    the interferometer (3) takes the form of a fibre-optic interferometer.

5.  System according to one of claims 1 to 4,
    **characterized in that**
    the reflection of the retina (6) passes via an optical imaging device (30) to a device (9) for detecting the aberrations of the wave fronts in mydriasis of the eye (2).

6.  System according to claim 5,
    **characterized in that**
    the device for detecting the aberrations of the wave fronts in mydriasis of the eye (2) takes the form of a Hartmann-Shack sensor (9).

7.  System according to one of claims 1 to 6,
    **characterized in that**
    the reflector (16) is moved measurably in its linear position by means of a drive device (18).

8.  System according to one of claims 1 to 7,
    **characterized in that**
    the optically at least partially transparent elements take the form of cylinders (22) made of polymethylmethacrylate, i.e. PMMA.

9.  System according to claim 8,
    **characterized in that**
    the cylinders (22) are provided on a wheel (19) that is disposed at least approximately at right angles to the optic axis of the reflector.

10. System according to claim 9,
    **characterized in that**
    in each case one of the cylinders (22) on the wheel (19) is selectively rotatable in the manner of a revolver cylinder into the light path.

11. System according to claim 9 or 10,
    **characterized in that**
    the wheel (19) is driven by a stepping motor (23).

12. System according to claim 9, 10 or 11,
    **characterized in that**
    disposed on the wheel (19) is an alternating arrangement of apertures (20) and cylinders (22).

13. System according to one of claims 1 to 12,
    **characterized in that**
    a superradiant diode, i.e. an SRD, serves as light source (10).

14. System according to one of claims 4 to 13,
    **characterized in that**
    the light from one end of a fibre (14) of the interferometer (3) passes via an optical imaging device into the eye (2).

15. System according to claim 14,

> **characterized in that**
> the imaging device comprises at least one lens (26).

16. System according to claim 14 or 15,
    **characterized in that**
    the imaging device comprises at least one diffractive optical element (27).

17. System according to claim 14, 15 or 16,
    **characterized in that**
    the imaging device comprises at least one beam splitter (28) for coupling the device (9) for detecting the aberrations of the wave fronts.

18. System according to claim 16,
    **characterized in that**
    the diffractive optical element (27) is designed in such a way that the first order of diffraction is focused onto the surface (4') of the cornea (4).

19. System according to one of claims 6 to 18,
    **characterized in that**
    the Hartmann-Shack sensor (9) comprises a lens array (34) and a detector field (35).

20. System according to claim 19,
    **characterized in that**
    the detector field (35) is formed by CCD sensors.

21. System according to one of claims 1 to 20,
    **characterized by**
    at least one electronic data processing device (46/24) for acquisition and evaluation of the measurement data as well as for open-loop/closed-loop control of the displacement path of the reflector (16).

## Revendications

1. Système de mesure sans contact de la qualité de reproduction optique d'un oeil (2) avec un interféromètre (3), dans lequel le système est aménagé de telle sorte qu'il réalise le procédé de mesure suivant : au moins une impulsion lumineuse est couplée dans l'oeil (2) avec une courte longueur de cohérence par une source de lumière (10), sachant que l'on fait varier la longueur de trajectoire optique d'au moins un bras de l'interféromètre (3) pour la mesure de la longueur (L) de l'oeil (2), jusqu'à ce qu'il se produise dans un détecteur (40) un motif d'interférence typique entre une réflexion de la cornée (4) et une réflexion de la rétine (6) de l'oeil (2), lequel, avec un tronçon de course (As) connu de la variation de la trajectoire optique, permet des conclusions sur la longueur (L) de l'oeil (2), sachant que la variation de la longueur de trajectoire optique s'effectue par l'introduction de premiers éléments (22) optiquement au moins partiellement transparents, et par au moins un deuxième élément (16) de l'interféromètre, pouvant être déplacé de façon définie dans l'au moins un bras (17) respectivement de l'interféromètre (3), et sachant que la longueur optique du premier élément (22) au moins partiellement transparent est adaptée à la longueur optique de l'oeil (2).

2. Système selon la revendication 1,
   **caractérisé en ce que** l'élément de l'interféromètre (3) pouvant être déplacé de façon définie est configuré comme réflecteur (16).

3. Système selon la revendication 2,
   **caractérisé en ce que** le déplacement (25) du réflecteur (16) et l'introduction de l'élément (22) optiquement au moins partiellement transparent s'effectue dans le chemin des rayons du même bras (17) de l'interféromètre (3).

4. Système selon la revendication 1, 2 ou 3,
   **caractérisé en ce que** l'interféromètre (3) est configuré comme interféromètre à fibres optiques.

5. Système selon l'une des revendications 1 à 4,

**caractérisé en ce que** la réflexion de la rétine (6) accède, en mydriase de l'oeil (2), par un dispositif d'imagerie optique (30) à un dispositif (9) pour la saisie de l'aberration des fronts d'ondes.

6. Système selon la revendication 5,
**caractérisé en ce que** le dispositif pour la saisie de l'aberration des fronts d'ondes en mydriase de l'oeil (2) est configuré comme capteur de Hartmann-Shack (9).

7. Système selon l'une des revendications 1 à 6,
**caractérisé en ce que** le réflecteur (16) est déplacé de façon mesurable dans sa position linéaire au moyen d'un dispositif d'entraînement (18).

8. Système selon l'une des revendications 1 à 7,
**caractérisé en ce que** les éléments optiquement au moins partiellement transparents sont configurés comme cylindres (22) en poly(méthacrylate de méthyle), c'est-à-dire en PMMA.

9. Système selon la revendication 8,
**caractérisé en ce que** les cylindres (22) sont montés sur une roue (19) disposée au moins à peu près perpendiculairement à l'axe optique du réflecteur.

10. Système selon la revendication 9,
**caractérisé en ce que** l'un respectivement des cylindres (22) sur la roue (19) peut être basculé au choix dans le trajet de la lumière à la manière d'un barillet de revolver.

11. Système selon la revendication 9 ou 10,
**caractérisé en ce que** la roue (19) est entraînée par un moteur pas à pas (23).

12. Système selon la revendication 9, 10 ou 11,
**caractérisé en ce que** des ouvertures de diaphragme (20) et des cylindres (22) sont disposés alternativement sur la roue (19).

13. Système selon l'une des revendications 1 à 12,
**caractérisé en ce qu'**une diode superélectroluminescente, c'est-à-dire une SLD, sert de source de lumière (10).

14. Système selon l'une des revendications 4 à 13,
**caractérisé en ce que** la lumière accède d'une extrémité d'une fibre (14) de l'interféromètre (3) dans l'oeil (2) via un dispositif d'imagerie optique.

15. Système selon la revendication 14,
**caractérisé en ce que** le dispositif d'imagerie optique comporte au moins une lentille (26).

16. Système selon la revendication 14 ou 15,
**caractérisé en ce que** le dispositif d'imagerie optique comporte au moins un élément optique diffracteur (27).

17. Système selon la revendication 14, 15 ou 16,
**caractérisé en ce que** le dispositif d'imagerie optique comporte au moins un diviseur de faisceau (28) pour le couplage du dispositif (9) de saisie des aberrations des fronts d'ondes.

18. Système selon la revendication 16,
**caractérisé en ce que** l'élément optique diffracteur (27) est configuré de telle sorte que le premier ordre de diffraction est focalisé sur la surface (4') de la cornée (4).

19. Système selon l'une des revendications 6 à 18,
**caractérisé en ce que** le capteur de Hartmann-Shack (9) comporte une matrice de lentilles (34) et un champ de détection (35).

20. Système selon la revendication 19,
**caractérisé en ce que** le champ de détection (35) est formé de capteurs CCD.

**21.** Système selon l'une des revendications 1 à 20,
**caractérisé par** au moins une unité électronique de traitement de données (46/24) pour la saisie et le dépouillement des données de mesure, ainsi que pour la commande/régulation de la course de déplacement du réflecteur (16).

Fig. 1

EP 1 223 848 B1

Fig. 2

Fig. 3

Fig. 4

corneal reflex

Fig. 5

Fig. 6